# EUROPEAN PATENT APPLICATION

(11) **EP 1 575 010 A1**
(43) Date of publication of application: **14.09.2005**
(21) Application number: 04380056.4
(22) Date of filing: 12.03.2004
(51) Int. Cl.: G08B 21/02, G08B 25/01

(54) **Detector and people-monitoring device for the provision of tele-assistance**

(71) Applicant: Ruiz-Morales Fadrique, Eugenio, 28230 Las Rozas, Madrid (ES); Mikuski, Francisco Paul, 28230 Las Rozas, Madrid (ES); Millas Rivas, Gonzalo, 28230 Las Rozas, Madrid (ES)
(72) Inventor: Ruiz-Morales Fadrique, Eugenio, 28230 Las Rozas, Madrid (ES); Mikuski, Francisco Paul, 28230 Las Rozas, Madrid (ES); Millas Rivas, Gonzalo, 28230 Las Rozas, Madrid (ES)
(74) Representative: Sanchez del Campo Gonzalez de Ubierna, Ramon

(57) **Abstract**

The device comprises a microcontroller module (1) to which the following are connected: a touch-sensitive panel module (2), a liquid crystal display module (3), an oscillator radio module (4), a GPS module (5), a radio communications module (6), a mobility module (7), a temperature and identification module (8), a skin resistivity detection and use sensor module (9), a power supply and battery charger module (10), a differential communication module (11), a GSM/GPRS module (12), an EEPROM memory module (13) and a voice digitization module (14). The device formed by said modules is small in size and can be worn by a person as a watch, pendant, etc. It is used to detect, monitor, locate and provide remote assistance to the wearer of the device, as well as generate help signals or emergency calls by the wearer himself. It is for particular use by people who are in danger of going outside certain geographical limits or by people suffering from memory lapses.

## Description

### OBEJCT OF THE INVENTION

The present invention relates to a small-sized device, designed as a detector and to provide a system for monitoring people, with means of controlling the vital parameters of said people and providing immediate tele-assistance services and remote medical supervision at a determined time.

The device can be in the form of a bracelet, wrist watch, pendant, necklace or other small object, and has the electronic means and suitable software for the whole unit to provide a service which monitors and protects the wearers who need to be located immediately in situations of danger or when said persons move outside certain set limits of a pre-programmed geographical area, making it especially useful for children or people suffering from illnesses involving memory lapses or from Alzheimer's, so that via the device any person can be monitored remotely, so that vital signs such as body temperature, ohmic skin resistance and body movement indices can be checked, the user himself also being able to generate signals for help or emergency calls at any time.

### BACKGROUND OF THE INVENTION

Although systems and equipment providing a service for monitoring people do exist, these systems or equipment are bulky, and cannot be carried around by those people in need, and systems or devices known are not known which can determine on their own whether any of the corresponding vital parameters, such as body temperature, ohmic skin resistance, body movement index, etc., move outside pre-set limits, i.e., there are no known small-sized commercial devices which are easy to carry about and which provide a service for monitoring people, and even less apparent are those which have means for monitoring the vital parameters of said people so that services of tele-assistance and remote medical supervision can be provided.

### DESCRIPTION OF THE INVENTION

The device recommended has been designed to resolve the problem disclosed above, since it can be considered as a miniaturized object which can be carried around by the user himself, as a watch, bracelet or pendant, etc., and having the capacity to activate a communications system that works, either through a GSM (Global System for Mobile Communications) or UHF (by radio) communications system, by activating a miniature GPS (Global Positioning System) inside the device itself, and sending to the service centres or any GSM mobile telephone, the position, in UTM coordinates (Universal Transverse Mercator), of the person wearing the device, together with the reason for raising the alarm, whether it be body temperature, ohmic skin resistance, body mobility, activation of an alarm or help button, or indeed if the person wearing the device is outside the geographical area pre-programmed in the device.

More specifically, the device of the invention includes a series of electronic modules and software designed to accompany it and provide the services mentioned above.

The modules will be electrically connected to a microcontroller, which is the main module, with low electricity consumption and having means for digitally and analogically monitoring all the systems or modules in the device.

In addition to the above microcontroller, the device has a miniature GPS module which can calculate the UTM coordinates in outside spaces and inside buildings, because it has high-extensibility means and advanced electronics, as well as having an antenna for capturing signals emitted by satellites.

It also includes a touch-sensitive panel module, comprising a glass film which acts as a touch-sensitive sensor with resistive characteristics, so that by using a four-contact connector with the appropriate specialized electronics and by using software included inside the microcontroller, said module permits the detection of the precise point or Cartesian coordinates when exerting any pressure on the surface of said sensor or panel, for which reason it permits the detection of points with a high-level of precision.

The purpose of said touch-sensitive panel module is to detect the pressure exerted by a finger or other object on the panel, to indicate that the request for assistance alarm has been made, also allowing the time and date display to be activated, which is shown in alphanumerical characters on the liquid crystal display of another module, also controlled by the microcontroller. The touch-sensitive panel module also provides a means, as with an alphanumerical keyboard, for selecting options, letters, sentences, icons or numbers, which are shown on the above liquid crystal display, and for accessing other device functions which can be selected by a number of options shown on said display.

The device includes another module which is a constituent element of the above liquid crystal display, which has a controller chip connected to the microcontroller, whose purpose is to show any alphanumerical character with different types of script, alphabet, size and colour, including icons, pictures and graphics. This module includes a cold-light generating panel, the electric power required for it to work being generated by a direct-current electrical power source of preferably 3.3 volts, to convert it into an alternating electric power of 200 volts, this electrical source being located in an electrical supply and battery charging module which will be described later.

Another module of the device comprises a real-time clock system, called RTC, which allows the time and date, as well as a perpetual calendar, to be stored in the memory of the microcontroller, with precision of one second per month, said RTC module being made up of an electronic circuit, designed for storing the time and date parameters mentioned above in its internal memory, including the perpetual calendar which can calculate and manage leap years, also serving as a means for generating pre-set alarms with date, hour, minutes and seconds, from the microcontroller, these parameters being presented as break pulses. Said RTC also generates a clock pulse with a suitable frequency for an oscillating signal used for the microcontroller to function.

Another part of this RTC module is a high-frequency oscillator of miniature crystal which supplies said clock signal to a GPS module, also included in the device, and to a radio transmit and receive module.

The device also includes the aforementioned GPS module, whose purpose is to capture and obtain the UTM localization coordinates via signals received from the array of satellites from the module or GPS system, this being miniature in size and highly sensitive for capturing satellite signals inside buildings, in tunnels, urban canyons, etc.

Said module is designed to carry out internal computations to calculate the correct UTM localization coordinates within a maximum time of four seconds.

It also includes electrical connections with the microcontroller module, so that it can be programmed, send resistance commands and obtain calculated information on position, direction of movement, height and other information specific to these systems.

The device also includes a programmable digital transmit and receive radio module, which can work in frequency bands of between 400 and 500 MHz and capable of transmitting digitized information in different formats or modulations, it has an internal antenna which can both transmit and capture radio signals in the specified frequency band, and also has electronics with a link for communicating digitally with other tele-medicine equipment.

It also has the electronics required for transmitting and receiving radio-electric signals, with a built-in antenna, a transmit and receive switch and software which, used in the microcontroller module, provides the remote correction of communication errors without the need for retransmission.

With this module, digitized voice signals from a voice digitization module can be transmitted to the corresponding charger of a device, the audio contained in the voice being sent internally by the device to said voice digitization module, from where it is sent to its charger where inverse electronics will reconstruct the audio and amplify it, retransmitting the audio by wireless means both to the charger equipment of the device and to the wireless headphones containing the appropriate electronics.

The device also includes a mobility module made up of a miniature accelerometer, fitted with acceleration sensors on the X and Y axes, which are parallel to the floor of its base, this module being designed to detect the mobility of the people wearing the device, and to detect any blows, impacts or heavy falls, so that the necessary alarms can be generated.

A temperature and identification module is also included, and this is made up of a digital thermometer with internal software and electronics for measuring body temperature, carrying out a dynamic comparison of the reading of the temperature calculated with a value for maximum internal temperature and one for minimum internal temperature, programmed and stored in a non-volatile memory, so that when it is connected to the module's internal power supply, it will emit an alarm signal to the microcontroller module if the body temperature of the person wearing the device exceeds the pre-set limits.

The digital thermometer built into this module includes an internal ROM (Read-Only Memory).

The device includes another module called the ohmic skin resistance detection and use sensor, which detects whether the device is being carried by the user or wearer, and measures the values of resistance of the device wearers' skin to electrical current, to determine situations of anguish, stress, perspiration and other unusual symptoms in the wearers' behaviour, which change the skin's electrical resistance value.

This module includes a signal amplifier, the signals being analysed by an analog-digital converter which forms part of the microcontroller module, so that using the appropriate software in the non-volatile memory of the microcontroller module, it can be checked whether the ohmic skin resistance detection and use sensor is in fact in contact with the wearer's or user's skin, allowing the approximate resistance of the skin to be determined at that time, comparing the values obtained with other resistance values obtained in the laboratory.

Said module also includes an oscillator and impedance transformation circuit, as well as an amplifier detector circuit, a comparator comprising an analog-digital compressor and an on/off circuit.

Another module of the device provides the power source to supply the electricity needed for all the modules which form the device, the power source comprising an electricity storage system made up of two lithium polymer ion batteries, and a protection system containing a diode to prevent a polarity reversal from damaging the electronics when charging the battery, and a Zener diode which is earthed to prevent the battery's charging voltage from exceeding a predetermined voltage of 8.2 volts, there also being a charging system, charging detection and charging suppression of the batteries, a 3.3 volt power supply with a voltage regulator, another power supply with a 4 volt voltage regulator, and a power supply to illuminate the liquid crystal display on the display module.

In addition to the above-mentioned modules, the device includes a communications module for establishing a wired digital communication from the microcontroller module to the outside, also including two special watertight connectors which are shared with the audio output to the headphones of the transmit-receive radio module. In addition to this telecommunications module, there is also a wireless GSM/GPRS module (Global System for Mobile Communications / General Packet Radio Services), which allows the sending of signals supplied by the mobile cellular telephone operators, and a serial connection to the microcontroller module, which controls voice, short messaging and data communication, and also a serial connection for upgrading software for said module, once the device has been manufactured.

It also includes an audio connection and an additional audio connection to a voice digitization module, via which the audio can be digitized and converted into data for retransmission via the transmit and receive radio module to the wireless headphones or to the charger equipment for reproducing said audio.

The aforementioned GSM/GPRS module, also includes a watertight microphone and an internal antenna which permits producing and transmitting the radio signals, software for operating the module and establishing and managing the wiring to the Internet®, provided via the mobile cellular telephone operators, as well as including software with all the TCP I/P routines (Transmission Control Protocol Internet/Protocol) which are needed to establish this type of connection.

Lastly, the device includes a non-volatile EEPROM memory module (Electrically Erasable Programmable Read-Only Memory) for storing software routines and data needed for the device to work, and a voice digitization module for retransmitting the voice received via the GSM module, so that the audio contained in the voice and received via GSM, is converted into data via a CODEX electronic circuit, digitizing said audio to send it to the microcontroller module, which only sends it to a UHF transceptor for its respective wireless transmission.

### DESCRIPTION OF THE DRAWINGS

To accompany the description given and help provide a better understanding of the characteristics of the invention, and in accordance with an example of a preferred practical embodiment thereof, a single page with drawings is attached as an integral part of said description which, in a purely illustrative and non-limitative way, represents a block diagram corresponding to the different modules making up the detector and monitoring device being the object of the invention.

### PREFERRED EMBODIMENT OF THE INVENTION

It can be seen in the said drawing how the device of the invention first comprises an ultra-low energy RISC (Reduced Instruction Set Computing) microcontroller module (1), which includes an analog comparator, a 10-channel analog-digital converter, an internal clock generator, 64 KB of non-volatile FLASH memory, 2 KB of internal RAM (Random Access Memory), the non-volatile FLASH memory block being shown with the reference (1').

Said microcontroller module (1) will always be switched on as long as the device has sufficient power in its internal batteries, and has two operating modes: one, using a 32,768 Hz low-frequency clock supplied by an RTC module and oscillator (4), the energy consumption of the microcontroller (1) being less than 30 microamperes. The other operating mode for said microcontroller module (1) controlled by software is by activating an 8 Mhz crystal connected directly to said microcontroller module (1), so that in this operating mode the microcontroller has a nominal consumption of less than 350 microamperes.

Said microcontroller module (1) together with the specially-developed software, controls and supervises the whole operation of the device, including all of its modules, which will be described later.

The device includes a touch-sensitive panel module (2), made up of a film of glass and transparent plastic material which forms a touch-sensitive sensor with resistive characteristics, and which is attached to the liquid crystal display with adhesive. This arrangement includes a four-contact connector (4), connected to non-specialized electronics and, using software included inside the microcontroller module (1), the precise point (Cartesian coordinates) at which any pressure is exerted (by a finger or object) on the surface of said sensor or panel, can be obtained.

Said sensor, together with the control electronics and microcontroller module (1), can detect points with a surface area of one millimetre squared and a precision of over 2 mm distance between one point and another.

Said sensor, which is the module (2) being described, is designed to carry out the following functions:
- To detect pressure exerted on the panel by a finger or object for raising the request-for-attention alarm. To activate this type of situation, constant pressure should be exerted for a period of more than 4 seconds on the surface of said sensor or panel.
- To activate the time and date display, shown in alphanumerical characters on the liquid crystal display on a display module (3). This device function will be activated by pressing on the surface of the sensor with a finger or other object, for a period of less than 2 seconds.
- To access other device functions which can be selected in an options menu shown on the display module (3). This is done by pressing the sensor again for a period of less than 2 seconds, once the time display option has been activated.
- To select, as with an alphanumerical keyboard, the options, letters, sentences, icons or numbers shown on the liquid crystal display of the module (3) by pressing with a finger or object on the character, sentence, letter, number or icon shown on the liquid crystal display.

The electronics for controlling this module (2) can be switched on or off electronically by the microcontroller module (1) together with its resident software and for the purpose of saving energy.

The device includes the above liquid crystal display module (3) made up of a tailor made liquid crystal display, which includes a controller chip connected to and controlled by the microcontroller module (1), so that any alphanumerical character with different types of script, alphabet, size and colour can be displayed, including icons, pictures and graphics.

Said display module (3) is designed and built to a specific size in accordance with the dimensions of the mechanics and external design of the device, and includes a white cold-light generating panel in its rear part, which uses the phenomenon of electroluminescence, with an electric potential of about 200 volts of alternating current in the electrical contacts of this illuminator. The electrical potential needed for this illuminator to work is generated by a power supply which uses a direct current of 3.3 volts to convert it into an alternating electrical current of 300 volts. The above power supply is located in an electrical supply and battery charging module (10), to be disclosed later, and the electrical activation or deactivation of this is controlled by resident software in the microcontroller (1).

There is another module called RTC and oscillator, which can be defined as a real-time clock, with the following functions:
- To store the time and date in its internal non-volatile memory, including a perpetual calendar which can calculate and manage leap years.
- To generate alarms, set with date, hour, minutes and seconds, from the microcontroller module (1), so that these alarms are shown as break pulses to the microcontroller itself (1).
- To generate a clock pulse with a frequency of 32,768 KHz as required for an oscillating signal used by the microcontroller module (1) to function and also used by a GPS module (5) for its internal operation.

In addition to the electronic part for carrying out the aforementioned functions described for the RTC, the RTC module and oscillator include an electronic part which consists of a miniature temperature-controlled crystal oscillator which supplies said clock signal to the module (5), and to the UHF radio communications module (6), as a frequency and references needed for tuning the receive and transmit radios which are found in these modules.

Said high-precision oscillator circuit is switched on and off via software in the microcontroller module (1) in order to save energy and will only be switched on when required.

The purpose of the GPRS module (5), referred to earlier, is to capture and obtain the UTM localization coordinates via the radio signals mentioned earlier from the satellite array of the GPRS system. Due to its high sensitivity, this module (5) can capture GPS satellite signals inside buildings, in tunnels and natural and man-made canyons.

Said module includes electrical connections to the microcontroller module (1) for programming it, after sending it resistance commands and obtaining from it the calculated information on positioning, direction of movement, height and other information typical of these systems.

Said module (5) also includes a half lambda long-wave antenna centred at 1,574 KHz with sufficient bandwidth to capture all the frequency waves in which the GPRS system operates.

This module (5) also functions in assisted time (GPRS) which means that, once switched on and duly assisted, it can carry out internal computations for correct calculation of the UTM localization coordinates within a maximum time of 4 seconds. This module is also designed so that, together with a different software from that used in the device being described, it can continue operating autonomously without the need for outside assistance.

There is also a UHF radio communications module (6), consisting of a narrow band transmit and receive digital radio, which can be programmed to operate in radio channels with frequencies of 12, 25, 50 and 125 KHz, respectively. This communications module (6) can be programmed to transmit and receive radioelectrical signals in a frequency band of between 400 MHz and 500 MHz, and with ASK (Amplitude Shift Keying), FSK (Frequency Shift Keying) or GFSK (Gaussian Frequency Shift Keying) modulation, depending on the programming.

Said communications module (6) has a radioelectrical sensitivity of -121 dBm when operating in 12 KHz broadband channels, and a programmable transmission power of between -20 and 10 dBm.

These electronics allow the device to transmit and receive digital information from the charging equipment also including the digital radio system. With this radio link, the device could communicate digitally with other telemedicine equipment and between those with cardio-pulmonary detection and analysis systems, blood oxymetry sensors, spyrometers and arterial pressure meters, all of which form part of the set of equipment included in this device.

The aforementioned UHF radio module (6) has all the necessary electronics for transmitting and receiving radioelectrical signals, and includes the antenna, transmit and receive switch and software used in the microcontroller module (1), allowing remote error correction without the need for retransmission, unless the corrupt digital information received is more than 30% of the correct digital information received. In the latter case, the software has the necessary detection mechanisms to activate retransmission of the blocks of missing data.

Via this same module (6), the digitized voice from a voice digitization module (14) can be transmitted to the device charger so that the audio contained in the voice received by a GSM and GPRS module which will also be explained later, is sent internally by the device to the voice digitization module (14), where it is converted into binary blocks which are transmitted by the module (6) to its charger, and where reverse electronics will reconstruct the audio and amplify it by some 3 watts so that it can be listened to with the appropriate volume in a loud speaker outside the charger, a loud speaker which can be connected to the charger equipment of the device via a female RCA audio connector, available for this purpose in the casing of the charger.

This digital radio link can be used to transmit audio (voice) received by the GSM and GPS module (12) at a distance, so that this audio can be retransmitted by wireless means both to the charger equipment of the device and to the wireless headphones which contain the appropriate electronics.

The device also includes a mobility module (7), made up of a miniature accelerometer fitted with acceleration sensors in the X and Y axes, parallel to the floor of its base. This module has the suitable electronics for detecting the mobility of the persons using the device, detecting the movements of the two coordinates' axes and translating them into an electrical voltage to represent the instantaneous movement of the arm and body of the person in question.

Both voltages are added together and quantified in an analog-digital converter located inside the microcontroller module (1), the latter including special software which calculates the mobility of the person, memorizing the values corresponding to the normal patterns of mobility of the users during their first month of use (learning period), and then compares the values determined in those readings made by the system several times a day and thus determining the periods of excess mobility or minimum mobility with the corresponding alarms being generated. Excess mobility or lack of mobility in human beings are natural signs which are directly related to different types of illnesses and pathologies.

This mobility module (7) is also designed to detect any blows, impacts and heavy falls suffered by the user, as well as detecting periods of unconsciousness or minimum mobility, so that alarms can be generated in cases of periods of unconsciousness caused by a blow.

The electronics used in this system or module (7) are accompanied by resident software in the microcontroller module (1), designed and developed to carry out this operation.

There is another module (8), called the temperature and identification module, made up of a digital thermometer with internal software and electronics for carrying out the following functions:
- Measure body temperature with a precision of 0.5 °C.
- Dynamically compare the temperature readings calculated with a value for maximum and one for minimum internal temperature, programmed and stored in the non-volatile memory of this electronic element so that, when connected to the internal power supply of the device, it will send an alarm signal to the microcontroller (1) if the users' body temperature exceeds the pre-set limits.
- The digital thermometer used in the device includes, in its internal ROM, a 64 bit binary number, which is unique and not repeated and represents the identity of each device. This number can be extracted from inside the digital thermometer and inside the microcontroller module (1) by means of a software routine, implemented for this purpose for the secure identification of each device.

The digital thermometer of said module (8) functions via software designed to have the functionalities described and which is resident in the non-volatile memory of the microcontroller module (1), including electronics designed to activate and deactivate the thermometer, to save on energy consumption.

The device also includes an ohmic skin resistance detection and use sensor module (9), designed to carry out the following functions:
- To detect if the device is being worn by the user.
- To measure the values of resistance to the electrical current of the skin of the person wearing the device, to determine situations of anguish, stress, perspiration and other unusual symptoms in the wearer's behaviour, which change the skin's electrical resistance value.

Said module (9) includes a flexible printed circuit termination which is back-to-back with the inside part thereof so that the electrical contacts are in contact with the skin. This extended part includes three gold-plated conductors, with the central conductor generating a very low-voltage electrical pulse, allowing the alternating voltage generated to be picked up by the other two electrical conductors on the ends connected to a high-gain and low-noise instrumental amplifier.

The amplified signals are analysed by an analog-digital converter in the microcontroller module (1) so that, by means of resident software in the non-volatile memory of the latter, it can be identified if the device is in contact with the user's skin and, in the same way, the approximate resistance of the skin at that time can also be determined, comparing the values obtained with other resistance values obtained in the laboratory.

Said module (9) includes an oscillator and impedance transformation circuit, as well as an amplifier detector circuit, a comparator made up of an analog-digital converter, integrally resident in the microcontroller module (1), and another on/off system of said electronics for reducing the internal electronic consumption of the device, the whole operation of the module being controlled by the software designed for such purpose and which is resident in the non-volatile memory of the microcontroller module (1).

The device also includes a module corresponding to a power supply (10), comprised of several electronic circuits connected to the internal power supply required for all the modules included in the device, the power supply comprising the following parts:
- Electrical storage system made up of two lithium polymer ion batteries arranged in series and with an individual storage capacity of 200 miliamperes, at a normal working voltage of 3.7 VDC.
- Back-up system made up of a diode to prevent a polarity reversal from damaging the electronics of the device when charging the batteries. A Zener diode with a nominal voltage of 8.2 volts is also included, and is earthed to prevent the battery charging voltage from exceeding said voltage.
- Charging system, charging detection and charging supervision of the batteries, comprised of a voltage supervision voltage at the battery charger input, this socket being connected to one of the multiplexed channels of the analog-digital converter of the microcontroller module (1) to detect if the device is charging, if the charging voltage is correct and if the process for charging the batteries has finished.
- 3.3 volt power supply with an integrated voltage regulator and fitted with input and output filters, supplied with nominal voltage generated by the batteries, set at a working voltage of 7.2 volts.
- Power supply for the GSM and GPRS module (12), which has a voltage regulator with a direct current capacity of 500 miliamperes and adjustable 4 volt output, including input and output filters, two serial-connected super-capacitators, providing a charging capacity of 0.3 faradays and 4 volts of working voltage, a balance circuit for the two super-capacitators with an intrinsic energy consumption of less than 10 microamperes, so that the two super-capacitators always maintain a balanced charge. This power supply provides a constant consumption of 50 miliamperes and a capacity to supply impulses of more than 2 amperes per time period of up to 500 thousands of a second, which is required for the proper functioning of the module (12).
- Power supply for the illuminator of the liquid crystal display module (3), this supply being designed so that a supply of only 3.3 volts of direct current generates between 70 and 100 watts of alternating current, so that the voltage generated is sufficient for the electro-luminescent film in the liquid crystal display base to generate light. Said supply is switched on and off by a low energy activation circuit controlled by the microcontroller module (1).
- Two watertight electrical connections which permit the device array to be connected to its charging equipment or charger.

The device also includes a differential communication module (11), designed to establish a wired digital communication from the microcontroller module (1) to the outside, for example, with the device's charger. The electronics of this communication module (11) include an electronic circuit specializing in this type of communication using a dual-wired connection.

These electronics ensure a secure communication for distances of over 10 metres at a speed which can exceed 500 Kbits per second.

The module (11) includes two special watertight connectors, which are shared with the audio output to the headphones also included in the device, and has a special feature in that the electronics of said module (11) provide protection against static current of up to 8,000 volts.

Said GSM/GPRS module (12), which forms part of the device of the invention, is forms a wireless communications module for communication via the GSM and GPRS system supplied by the mobile cellular telephone operators, and comprises:
- An internal serial connection to the microcontroller module (1), to control the voice, short messaging and data communication, it being a serial connection for upgrading the internal software for this module once the devices have been manufactured.
- An audio connection, received by the GSM system to the two connectors provided for its external headphones and which are shared with the differential communication module (11).
- An additional audio connection to the voice digitization module (14), via which the audio received in a GSM connection could be digitized and converted into data for retransmission by the radio communications module (6) to wireless headphones or to the charging equipment for reproducing said audio with sufficient volume, using an external loud speaker.
- A watertight microphone, which can be submerged up to a depth of 3 metres without being damaged, so that the user of the device can be heard speaking just like on any mobile telephone.
- A dual and triple-band miniature internal antenna is used by the GSM/GPRS system to receive and transmit the radio signals in which said communications system operates.
- Software to enable said module (12) to operate, this software residing in the microcontroller module (1) and comprising an array of AT commands and software in resident assembly language, both in the non-volatile memory of the microcontroller module (1). This software enables the module (12) to operate so that it can make and take incoming calls, send and receive short messages, in addition to other functions, etc.

In order to make said module (12) operative, the GPRS module with its capacity to establish and manage the wiring with the Internet® provided by the mobile cellular telephone operators, also includes software with all the TCP I/P and PPP (Point-to-Point Protocol) routines needed to establish this type of wiring.

The device also includes an I2C EEPROM memory module (13) with a capacity of 500 Kbits, able to store software routines and data required for the equipment to function.

Lastly, the device includes a voice digitization module (14) referred to earlier, whose purpose is to retransmit the voice received by the module (12) to other remote devices, using the UHF link. The audio contained in the voice, received by GSM, is converted into data via a CODEX electronic circuit, digitalizing said audio at an approximate rate of 8 Kb/second.

Once obtained, the digitized audio is sent to the microcontroller module (1), which then sends it to the UHF transceptor for its respective wireless transmission.

The block diagram of the modules described, also refers to the internal GPS antenna (15), the internal UHF antenna (16) of modules (5) and (6), the sensor element (17) connected to the module (9), the charger (18) connected to the power supply (10), with the corresponding charging connector (19), the array of batteries (20), a solar panel (21), a set of headphones and data (22) with the connector (23) to the differential communication module (11), a voice antenna (24) connected to the module (12), an audio output (25) between this module (12) and the voice digitization module (14), and a watertight audio microphone (26) connected to the GSM and GPRS module (12).

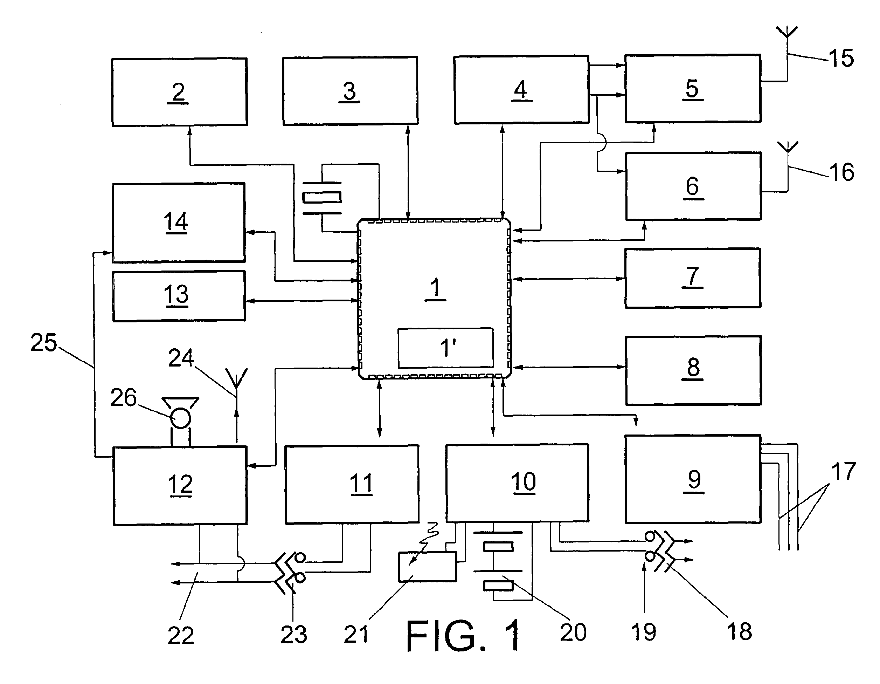

## Claims

1. Detector and people-monitoring device for the provision of tele-assistance which, being able to be in the form of a bracelet, wrist watch, pendant, etc., to be carried with or worn by the person, and being applicable for use for remote monitoring, location and protection of the person wearing it at all times, is **characterized in that** it comprises a plurality of electronically-connected modules with a low-energy microcontroller module (1), which includes an analog comparator, an analog-digital converter, an internal clock generator, a non-volatile FLASH memory and a RAM memory; the modules comprising the device corresponding to: a module called a touch-sensitive panel (2), formed by a touch-sensitive sensor with resistive characteristics, for indicating when a request-for-attention alarm has been made, and for activating the time and date display; a display module (3) formed by a liquid crystal display to show any alphanumerical characters, including a white cold-light generating panel; a module (4) defined as a real-time clock, for storing the time, date and a perpetual calendar in its non-volatile memory, and to generate pre-programmed alarms from the microcontroller module (1) and to generate a clock pulse, and forming part of this module (4), a temperature-controlled crystal oscillator; a GPS module (5) for capturing and obtaining UTM localization coordinates, via radio signals received from the array of satellites from the GPS module itself; a programmable digital radio transmit and receive module (6), which is a UHF radio communications module, which works in frequency bands between 400 MHz and 500 MHz, and having an internal antenna for transmitting and capturing radioelectrical signals in the band frequency mentioned; a mobility module (7) for detecting and memorizing the average mobility of the wearer of the device, and for determining electronically the anomalous changes in such mobility, also being able to detect strong blows caused by an accident or fall, generating alarms in the event of any such anomalous situations; a temperature identification module (8) for measuring body temperature, and for carrying out the dynamic comparison of the temperature readings calculated with a maximum and minimum internal value, programmed and stored in a non-volatile memory, emitting an alarm signal to the microcontroller module (1) when the temperature measured exceeds the pre-set limits; a skin deactivation detection and use sensor module (9), to detect if the device is being worn by the wearer, and to measure the values of resistance to electrical current of the skin of the person wearing the device, for determining situations of anguish, stress, perspiration and other unusual symptoms in the wearer's behaviour, sending an alarm to the microcontroller module (1) if the user is not wearing the device or, if the skin's ohmic resistance values detected are outside the pre-set limits; a module (10) or electrical power supply, with an electrical storage system made up of two batteries (20), with a protection system made up of a diode to prevent a polarity reversal from damaging the electronics, and a Zener diode which is earthed to prevent the batteries' charging voltage (20) from exceeding a determined voltage, there also being a charger or charging system (18), and charging detection and charging supervision of the batteries, with a 3.3 volt power supply with a voltage regulator, and a 4 volt power supply also with a voltage regulator for a GSM and GPRS module (12), and another power supply of the illuminator of the liquid crystal display module (3); a differential communication module (11) for establishing a wired digital communication between the microcontroller module (1) and the outside; a wireless communications GSM/GPRS module (12), for communications via the GSM and GPRS system supplied by the mobile cellular telephone operators, for sending and receiving short messaging, and for the digital connection to an Internet® network via the GPRS communications system; an EEPROM memory module (13) for storage and software routines and data needed for the device to work, and a voice digitization module (14) for transmitting the voice received by the GSM module (12) to other remote devices, using the UHF link.

2. Detector and people-monitoring device for the provision of tele-assistance, according to claim 1, **characterized in that**, to function, the microcontroller unit (1) uses a low-frequency clock supplied by the oscillator clock module (4), also being able to operate under the control of software via the activation of a crystal connected directly to the microcontroller module (1).

3. Detector and people-monitoring device for the provision of tele-assistance, according to claim 1, **characterized in that** the corresponding touch sensor of the touch-sensitive panel module (2), is made up of a film of glass and transparent plastic material, designed to detect pressure exerted on it, for a period of more than 4 seconds, to indicate that the request-for-attention alarm has been raised, and to activate the time and date display which is shown in alphanumerical characters on the liquid crystal display of the module (3), said function being activated by pressing on the surface of the sensor for a period of less than 3 seconds, said sensor module (2) allowing options or letters, sentences, icons or numbers represented on the display to be selected, by pressing the character, sentence, letter, number or icon shown on said liquid crystal display.

4. Detector and people-monitoring device for the provision of tele-assistance, according to claim 1, **characterized in that** the liquid crystal display module (3) includes a controller chip connected to and controlled by the microcontroller module (1), so that, as well as alphanumerical characters, icons, pictures and graphics can also be shown; the electric power for the cold-light generating panel of said display module (3) being generated by the power supply of the module (10), the electrical activation and deactivation thereof being controlled by software in the microcontroller module (1).

5. Detector and people-monitoring device for the provision of tele-assistance, according to claim 1, **characterized in that** the oscillator clock module (4) comprises two electronic circuits for generating the clock functions and the oscillator functions, the generation of a clock pulse being supplied by said module (4) as a signal required for the oscillator, and used by the microcontroller module (1) to function, and by the GPS module (5) for its internal operation.

6. Detector and people-monitoring device for the provision of tele-assistance, according to claim 5, **characterized in that** the liquid crystal oscillator of the clock module (4) supplies the clock signal to the GPS module (5) and the UHF radio communications module, as reference frequencies needed for tuning the receive and transmit radios in said modules.

7. Detector and people-monitoring device for the provision of tele-assistance, according to claim 1, **characterized in that** the GPS module (5) includes the appropriate connections with the microcontroller module (1) to allow it to be programmed, as well as the transfer of resistance commands and to obtain the calculated information on position, direction of movement, height and other information typical of these types of system; said GPS module (5) also having an antenna (15) for capturing frequencies.

8. Detector and people-monitoring device for the provision of tele-assistance, according to claim 1, **characterized in that** the UHF radio communications module (6) includes communications equipment for transmitting and receiving the digital information from charger equipment in the module itself, also enabling it to communicate with another telemedicine system; said module (6) including an antenna (16) and a transmit and receive switch which, when combined with the software used in the microcontroller module (1), enables the remote correction of communication errors without the need for retransmission; with the special feature that, via said module, the digitized voice from the voice digitization module (14) of the device is sent to the charger (18), the audio contained in the voice received by the GSM and GPRS module being sent internally to the voice digitization module itself, via the audio output (25).

9. Detector and people-monitoring device for the provision of tele-assistance, according to claim 1, **characterized in that** the mobility module (7) is made up of an accelerometer fitted with acceleration sensors on the X and Y axes, parallel to the floor of its base.

10. Detector and people-monitoring device for the provision of tele-assistance, according to claim 1, **characterized in that** the temperature and identification module (8) is made up of a digital thermometer connected to special electronics and the corresponding software for measuring, comparing readings and emitting alarm signals.

11. Detector and people-monitoring device for the provision of tele-assistance, according to claim 1, **characterized in that** the signals provided by the skin resistivity detection and use sensor module (9), are analysed by an analog-digital converter in the microcontroller module, as well as an oscillator and impedance transformation circuit, an amplifier detector circuit and a comparator made up of an analog-digital converter.

12. Detector and people-monitoring device for the provision of tele-assistance, according to claim 1, **characterized in that** the electrical power supply (10) includes electronic circuits and software for supervising the replacement process of the electric charge of the two batteries (20), including the transmission and reception of data between the microcontroller module (1) and a controller included in the charging system (18) of the module (10), so that said batteries (20) can be charged to the maximum.

13. Detector and people-monitoring device for the provision of tele-assistance, according to claim 1, **characterized in that** the differential communication module (11) includes two special connectors (23), which are watertight and shared with the audio output to the corresponding headphones (22) of the device.

14. Detector and people-monitoring device for the provision of tele-assistance, according to claim 1, **characterized in that** the GSM and GPRS module (12) includes an internal serial connection to the microcontroller module (1) for controlling voice, short messaging and data communication via an antenna (24), as well as another serial connection for upgrading the internal software of said module; there is also an audio connection received by the GSM system to the respective connectors provided for the external headphones (22), which are shared by the communications module which makes up the EEPPROM memory (13), the GSM/GPRS modules (12) having a watertight microphone (26) and the internal antenna (24), for receiving and transmitting the radio signals, as well as software with all the necessary routines for establishing the connection with the Internet® and software for making calls, and sending and receiving short messaging.

15. Detector and people-monitoring device for the provision of tele-assistance, according to claim 1, **characterized in that** the voice digitization module (14) includes an electronic circuit for converting the audio contained in the voice into data, digitalizing the audio for sending to the microcontroller module (1), from where it is then sent to a UHF transceptor for wireless transmission.
